# EUROPEAN PATENT APPLICATION

(11) **EP 3 040 123 A1**
(43) Date of publication of application: **06.07.2016**
(21) Application number: 14841214.1
(22) Date of filing: 21.05.2014
(51) Int. Cl.: B01J 35/02, A01N 55/02, A01P 1/00, A61L 2/08, A61L 2/16, B01D 53/86, B01J 31/04, B01J 37/03, B01J 37/04

(54) **PHOTOCATALYST USING REDUCING ORGANIC COMPOUND**

(30) Priority: 28.08.2013 JP 2013176411
(71) Applicant: National Research and Development Agency National Agriculture and Food Research Organization, Tsukuba-shi Ibaraki 305-85171 (JP)
(72) Inventor: MORIKAWA Claudio Kendi, Tsu-shi Mie 5142392 (JP); SHINOHARA Makoto, Tsu-shi Mie 5142392 (JP)
(74) Representative: Gille Hrabal
(86) International application number: PCT/JP2014/063429
(87) International publication number: WO 2015/029509

(57) **Abstract**

An object is to provide an inexpensive photocatalyst that can be used for degradation of organic substances or sterilization, can be used in any situations, is highly safe, and absorbs light having a wide range of wavelengths including visible light to exhibit an activity. A photocatalyst including, as an active component, a reaction product obtained by mixing a reducing organic substance having iron-reducing ability or a feedstock for supplying the reducing organic substance with an iron-supplying source in the presence of water is provided; specifically, a photocatalyst in which the reaction product exhibits a photocatalytic activity when irradiated with light having a wavelength of ultraviolet light, visible light, or infrared light; an organic substance degradation method including bringing the photocatalyst into contact with an object to be degraded and irradiating the photocatalyst with light having a wavelength of ultraviolet light, visible light, or infrared light; and a sterilization method including bringing the photocatalyst into contact with an object to be sterilized and irradiating the photocatalyst with light having a wavelength of ultraviolet light, visible light, or infrared light.

## Description

### Technical Field

The present invention is directed to a technology relating to a photocatalyst using a reaction product of a reducing organic substance having iron-reducing ability and iron. The present invention is also directed to a technology relating to an organic harmful substance degradation method or sterilization method using the photocatalyst.

### Background Art

### · Related-art Photocatalyst Technology

The photocatalyst can be used for degradation of organic harmful substances, sterilization, or the like only by irradiation with light, and hence has had high social needs as a convenient and versatile technology.

As a substance exhibiting a photocatalytic activity, there are known, in addition to titanium oxide, metal compounds of tungsten, indium, vanadium, silver, molybdenum, zinc, gallium phosphide, gallium, and arsenic, and all of them are substances exhibiting photocatalytic activities only when irradiated with light having a wavelength of ultraviolet light, 400 nm or less. Of those, the photocatalysts other than titanium oxide have problems such as very high cost and strong toxicity, and hence are not in practical use. At this time, only titanium oxide is in practical use as a photocatalyst.

When titanium oxide absorbs ultraviolet light, the titanium oxide generates reactive oxygen species and exhibits a photocatalytic activity for degradation of organic substances, sterilization, or the like. Based on the effect, use of titanium oxide is being increased such that it is applied to an exterior wall to prevent dirt from attaching to the wall.

However, titanium oxide does not exhibit the photocatalytic activity when irradiated with visible light having a wavelength of 400 nm or more, and hence has the following problems. Titanium oxide cannot be used for, for example, sterilization or degradation in a living space in which only visible light, such as fluorescent light, can be used, and situations to which titanium oxide can be applied are limited.

Further, to realize the photocatalytic activity by visible light, technologies for incorporating impurities (doping) have been attempted (see, for example, Patent Literatures 1 and 2), but have problems with difficult processing technologies and very high costs. In addition, a photocatalyst produced by the doping technology has a very weak activity, and hence no such photocatalyst is in practical use.

Further, in the U. S., titanium oxide is a substance recognized as a carcinogen, and questions are raised about safety of titanium oxide itself. Accordingly, a situation in which titanium oxide can be used is very limited.

From such circumstances, development of an inexpensive photocatalyst that can be used in any situations, is highly safe, and exhibits an activity by visible light has been expected.

### Citation List

### Patent Literature

[PTL 1] JP 07-303835 A
[PTL 2] JP 2006-305532 A

### Summary of Invention

### Technical Problem

An object of the present invention is to solve the above-mentioned problems to provide an inexpensive photocatalyst that can be used for degradation of organic harmful substances or sterilization, can be used in any situations, is highly safe, and absorbs light having a wide range of wavelengths including visible light to exhibit an activity.

### Solution to Problem

The inventors of the present invention have made extensive investigations, and have found that a reaction product obtained by mixing a reducing organic substance having iron-reducing ability or a feedstock for supplying the reducing organic substance with an iron-supplying source in the presence of water has a strong photocatalytic activity. The inventors of the present invention have further found that the photocatalytic activity is an activity exhibited by irradiation with not only ultraviolet light but also visible light or infrared light. The inventors of the present invention have further found that the photocatalyst is stable as a substance and can be used repeatedly. It should be noted that the iron-reducing organic substance and the iron-supplying source that are raw materials for the photocatalyst are inexpensive and familiar substances and are raw materials highly safe for a human body and an environment.

The inventors of the present invention have also found that the photocatalyst can be used to achieve degradation of organic harmful substances and sterilization by irradiation with not only ultraviolet light but also visible light or infrared light.

The present invention has been made based on those findings.

That is, the invention according to a first aspect relates to a photocatalyst, including, as an active component, a reaction product obtained by mixing a reducing organic substance having iron-reducing ability or a feedstock for supplying the reducing organic substance with an iron-supplying source in the presence of water.

Further, the invention according to a second aspect relates to a photocatalyst according to the first aspect, in which the reducing organic substance includes a polyphenol and/or ascorbic acid.

Further, the invention according to a third aspect relates to a photocatalyst according to the second aspect, in which the polyphenol includes one or more compounds selected from the group consisting of chlorogenic acid, caffeic acid, tannic acid, and catechin, or a compound having one or more of the compounds selected from the group consisting of chlorogenic acid, caffeic acid, tannic acid, and catechin in its molecule.

Further, the invention according to a fourth aspect relates to a photocatalyst according to any one of the first to third aspects, in which the feedstock for supplying the reducing organic substance includes one or more plant bodies selected from the group consisting of a fruit, a seed, a stem and leaf, a bud, a flower, a root, and an underground stem, or a processed product of the plant body.

Further, the invention according to a fifth aspect relates to a photocatalyst according to any one of the first to fourth aspects, in which the feedstock for supplying the reducing organic substance includes roasted coffee beans, tea leaves, a squeezed fruit juice, or a plant dry distillation liquid.

Further, the invention according to a sixth aspect relates to a photocatalyst according to any one of the first to fifth aspects, in which the iron-supplying source is mixed so that a content of the iron-supplying source is from 0.1 part by weight to 10 parts by weight, in terms of weight of iron element, with respect to 100 parts by weight, in terms of dry weight, of the reducing organic substance or the feedstock for supplying the reducing organic substance.

Further, the invention according to a seventh aspect relates to a photocatalyst according to any one of the first to sixth aspects, in which the reaction product exhibits a photocatalytic activity when irradiated with light having a wavelength of ultraviolet light, visible light, or infrared light.

Further, the invention according to an eighth aspect relates to a photocatalyst according to any one of the first to seventh aspects, in which the photocatalyst exhibits an organic substance degradation action by the photocatalytic activity.

Further, the invention according to a ninth aspect relates to an organic substance decomposer, including the photocatalyst of any one of the first to eighth aspects.

Further, the invention according to a tenth aspect relates to an organic substance degradation method, including: bringing the photocatalyst of any one of the first to eighth aspects into contact with an object to be degraded; and irradiating the photocatalyst with light having a wavelength of ultraviolet light, visible light, or infrared light.

Further, the invention according to an eleventh aspect relates to a sanitizer, including the photocatalyst of any one of the first to eighth aspects.

Further, the invention according to a twelfth aspect relates to a sterilization method, including: bringing the photocatalyst of any one of the first to eighth aspects into contact with an object to be sterilized; and irradiating the photocatalyst with light having a wavelength of ultraviolet light, visible light, or infrared light.

### Advantageous Effects of Invention

The photocatalyst according to one embodiment of the present invention has a property of exhibiting an activity when irradiated with not only ultraviolet light but also visible light or infrared light. Accordingly, the present invention is expected to be used for various applications in which titanium oxide in the related art is hard to use. For example, the photocatalyst can be used in a usual indoor space.

Further, the photocatalyst according to the embodiment of the present invention uses, as the iron-reducing organic substance that is a raw material therefor, the polyphenol or ascorbic acid, and is hence highly safe for a human body and an environment. On the other hand, titanium oxide in the related art is a substance recognized as a carcinogen in the U.S., which prevents prevalence of the substance.

In view of this, the photocatalyst according to the embodiment of the present invention is expected to be used for various applications in which titanium oxide is hard to use.

Further, according to the embodiment of the present invention, it is possible to provide the excellent photocatalyst by a simple method using only inexpensive raw materials (such as an iron compound and a reducing organic substance contained in a plant body). In particular, when, for example, plant body extraction residues (such as coffee grounds and tea dregs), a plant dry distillation liquid (by-product of carbonization), and a squeezed plant juice (such as grape juice) are used as feedstocks for supplying the reducing organic substance, the photocatalyst can be produced particularly inexpensively. On the other hand, titanium oxide in the related art costs tens of thousands of yen per 10 mg and is a very expensive material.

In view of this, the photocatalyst according to the embodiment of the present invention is expected to be used as a technology for solving the problem of production cost of titanium oxide.

The photocatalyst according to the embodiment of the present invention is expected to be widely used for sterilization or for degradation of organic substances in wide fields of food, medicine, public health, agriculture, environmental cleanup, and the like.

### Brief Description of Drawings

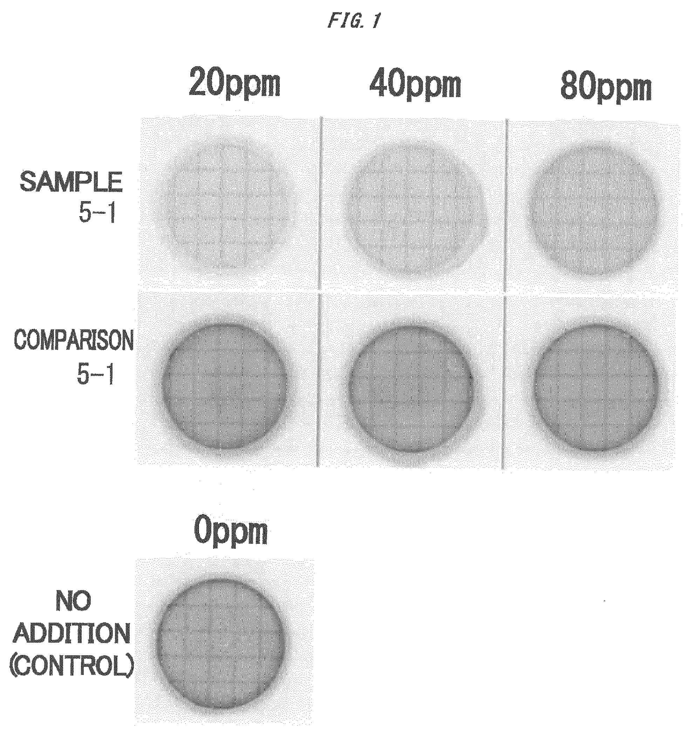

FIG. 1 is a photographic image for showing the results of a test of sterilization of *Escherichia coli* by irradiation with visible light in Example 5. In the photographs of the figure, the blue parts (black parts in black and white photographs) are parts in which *Escherichia coli* is present. The colorless parts are parts in which *Escherichia coli* is absent.

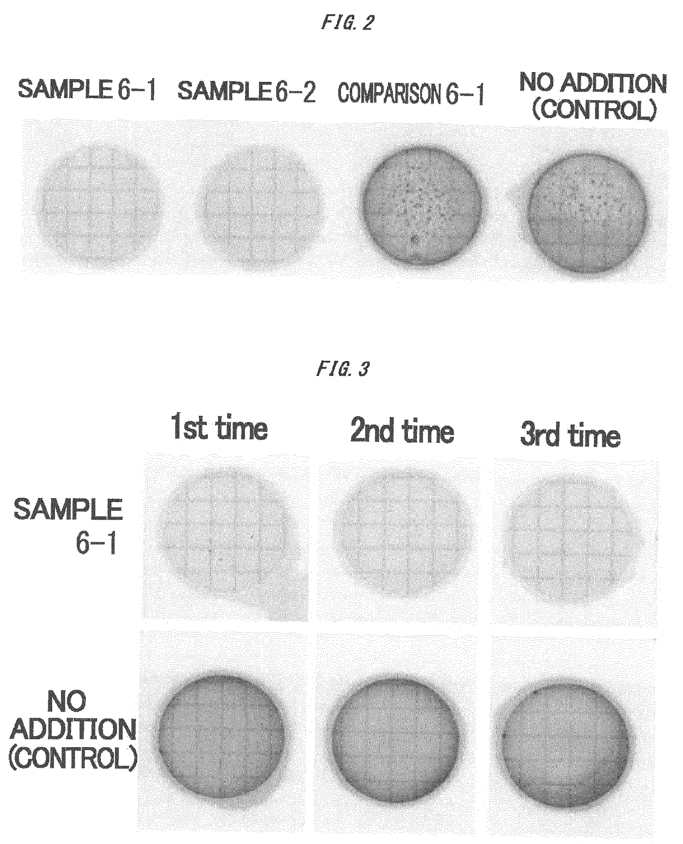

FIG. 2 is a photographic image for showing the results of a test of sterilization of *Escherichia coli* by irradiation with sunlight in Example 6. In the photographs of the figure, the blue parts (black parts in black and white photographs) are parts in which *Escherichia coli* is present. The colorless parts are parts in which *Escherichia coli* is absent.

FIG. 3 is a photographic image for showing the results of a test of sterilization of *Escherichia coli* by irradiation with sunlight in Example 7. In the photographs of the figure, the blue parts (black parts in black and white photographs) are parts in which *Escherichia coli* is present. The colorless parts are parts in which *Escherichia coli* is absent.

### Description of Embodiments

The present invention is directed to a technology relating to a photocatalyst using a reaction product of a reducing organic substance having iron-reducing ability and iron. The present invention is also directed to a technology relating to an organic substance degradation method or sterilization method using the photocatalyst.

### [Reducing Organic Substance]

In production of a photocatalyst of the present invention, a "reducing organic substance having iron-reducing ability" is used as a raw material. The organic substance can be defined as an organic substance having strong reducing power and having an action of reducing trivalent iron into divalent iron.

Specific examples of the organic substance may include ascorbic acid and polyphenols. Further, other than those compounds, a plant body or a processed product thereof may contain a large amount of a reducing organic substance having iron-reducing ability, and can be preferably used as a raw material of the present invention.

Herein, not only a free ascorbic acid but also ascorbate compound (such as potassium ascorbate and sodium ascorbate) may also be used as the 'ascorbic acid'.

In addition, the 'polyphenol' refers to a phenolic molecule having a plurality of hydroxy groups. The phenolic molecule is a compound contained in most plants, and various kinds thereof, such as flavonoids and phenolic acids, are known. Specific examples of the compound include catechins (such as epicatechin, epigallocatechin, epicatechin gallate, and epigallocatechin gallate), tannic acid, tannin, chlorogenic acid, caffeic acid, neochlorogenic acid, cyanidin, proanthocyanidin, thearubigin, rutin, flavonoids (such as quercitrin, anthocyanin, flavanone, flavanol, flavonol, and isoflavone), flavone, chalcones (such as naringenin chalcone), xanthophyll, carnosic acid, eriocitrin, nobiletin, tangeretin, magnolol, honokiol, ellagic acid, lignan, curcumin, coumarin, catechol, procyanidin, theaflavin, rosmarinic acid, xanthone, quercetin, resveratrol, gallic acid, and phlorotannin. In addition, examples thereof may also include compounds each having in its molecule one or more of those compounds (such as a composite that is a macromolecule obtained by bonding in such a manner that any of those compounds is contained).

Further, a polyphenol composition extracted from a fruit may be referred as polyphenol attached to the name of the fruit. For example, a polyphenol composition extracted from grapes is referred to as grape polyphenol.

In the present invention, a purified product of any of the above-mentioned compounds is preferably used as the raw material to enhance the activity of the photocatalyst.

### · Feedstock for Supplying Reducing Organic Substance

In the present invention, a plant body or a processed product thereof containing a polyphenol and/or ascorbic acid can be used as a feedstock for supplying the reducing organic substance.

Herein, examples of the plant body may include plant bodies each derived from one or more selected from the group consisting of a fruit, a seed, a stem and leaf, a bud, a flower, a root, and an underground stem.

Examples of the plant body raw material containing a large amount of the 'ascorbic acid' may include tomato, green pepper, red pepper, was gourd, bitter gourd, zucchini, cucumber, podded pea, pumpkin, eggplant, green pea, broad bean, green soybean, okra, acerola, citrus fruits (such as lemon, lime, orange, grapefruit, navel orange, Yuzu, kumquat, Kabosu, Watson pomelo, Hassaku orange, Iyokan, lime, satsumamandarin, Shekwasha, and mandarin), persimmon, kiwi fruit, papaya, blackberry, blueberry, cranberry, raspberry, bilberry, huckleberry, strawberry, melon, apple, pear, European pear, fig, peach, Japanese plum, guava, grape, prune, Akebi, durian, pineapple, mango, banana, cherry, pomegranate, watermelon, oleaster, loquat, cassis, chestnut, lychee, ginkgo nut, olive, avocado, tea, lettuce, cabbage, kale, mustard, potherb mustard, Japanese mustard spinach, radish, turnip, rape blossom, Chinese cabbage, qing-geng-cai, Takana, turnip greens, molokheiya, green onion, wild rocambole, garlic, cibol, leek, onion, shallot, perilla, Ashitaba, malabar spinach, watercress, asparagus, basil, water dropwort, celery, parsley, spinach, crown daisy, bamboo shoot, broccoli, cauliflower, sweet potato, potato, yam, lotus root, turnip, radish, brussels sprouts, and seaweeds (such as laver, wakame seaweed, kelp, and sea lettuce).

In addition, examples of the plant body raw material containing a large amount of the 'polyphenol' may include grape, strawberry, blackberry, blueberry, cranberry, raspberry,bilberry, huckleberry, apple, Japanese apricot, peach, Japanese plum, pear, European pear, cherry, citrus fruits (such as lemon, lime, orange, grapefruit, navel orange, Yuzu, kumquat, Kabosu, Watson pomelo, Hassaku orange, Iyokan, lime, satsuma mandarin, Shekwasha, and mandarin), loquat, kiwi fruit, mango, mangosteen, sweet pepper, prune, persimmon, banana, melon, dragon fruit, coffee, mulberry, Chinese matrimony vine, cassis, cashew, viburnum, guava, pomegranate, olive, assai palm, aronia, eggplant, tomato, grape, cacao, soybean, black soybean, adzukibean, green bean, peanut, black-seeded sesame, buckwheat, tartary buckwheat, coffee beans, sesame, red cabbage, tea leaves, sumac, Chinese sumac, crown daisy, broccoli, sugar cane, spinach, Japanese mustard spinach, Japanese honewort, okra, butterbur, onion, molokheiya, crown daisy, garlic, red onion, asparagus, parsley, eucalyptus, coffee, ginkgo, mint, udo, papaya, perilla, *Gymnema sylvestre*, senna, dandelion, field horsetail, ferns (such as bracken and osmund), banana, persimmon, oak, sawtooth oak, maple, sequoia, dawn redwood, pine, Japanese cedar, Japanese cypress, acacia, Japanese mallotus, *Gamblea innovans, Hydrangea macrophylla* var. *thunbergii,* akebi, *Acanthopanax spinosus,* Japanese clethra, anise magnolia, kobus magnolia, tara vine, *Lindera triloba, Lindera umbellata, Eleutherococcus sciadophylloides,* harlequin glory bower, Japanese bigleaf magnolia, silver vine, eucalyptus, guava, giant crape-myrtle, rooibos, dogbane, mulberry, Chinese matrimony vine, kudzu, Nikko maple, Bornean ironwood, merbau, Chinese parasol tree, sappanwood, brazilwood, melinjo, peach, cherry, magnolia, yerba mate, *Kandelia obovata,* blackmangrove, redmangrove, apple mangrove, nipa palm, grey *mangrove, Lumnitzera racemosa* Willd., looking-glass mangrove, burdock, sweet potato, purple sweet potato (sweet potato containing a large amount of purple dye), potato, yam, taro (such as *Colocasia esculenta* (L.) Schott or *Colocasia esculenta* f. ebiimo Makino), turmeric, lotus root, konjac, and seaweeds (such as laver, wakame seaweed, kelp, sea lettuce, arame, and *Eisenia arborea).*

The feedstock for supplying the reducing organic substance may be prepared by processing a plant body into, for example, a dried product, a squeezed juice, or an extract (in particular, a water or hot water extract, an alcohol extract, or a water-containing alcohol extract). In addition, the squeezed juice or extract may further be processed into a dried product before use.

To process the feedstock into a dried product, it is desired to perform a treatment such as fracture, pulverization, or powderization. Further, from the viewpoint of reaction efficiency with iron, it is preferable to process into powder having small particle diameters.

For ascorbic acid, water is preferably used as a solvent used for extraction. For polyphenols, water, hot water, ethanol, or water-containing ethanol is preferably used.

Further, a residue obtained by subjecting a plant body or a processed product thereof to extraction with water or hot water may also be preferably used as the feedstock for supplying the reducing organic substance.

Further, a dry distillation liquid obtained by thermal degradation of a plant body or a processed product thereof in a reducing condition (plant dry distillation liquid) may also be preferably used.

### · Plant body-derived Raw Material Advantageous in Raw Material Cost

In the present invention, when any of a squeezed fruit juice, a squeezed stem and leaf juice, a plant dry distillation liquid, roasted coffee beans, and tea leaves is used as the feedstock for supplying the reducing organic substance, the photocatalyst can be produced more inexpensively, which may provide an economically advantageous effect.

### (a) Squeezed Fruit Juice

The 'squeezed fruit uice' is preferably used as the feedstock for supplying the reducing organic substance. With regard to the kind of fruit to be squeezed, the fruits described in the foregoing paragraphs may be preferably used. In particular, a fruit rich in total polyphenols is preferred from the viewpoint of potency. Further, from the viewpoint of the cost of the raw material, squeezed juices of, for example, a grape, a banana, an apple, a persimmon, a tomato, and citrus fruits are preferably used.

### (b) Squeezed Stem and Leaf Juice

The 'squeezed stem and leaf juice' is preferably used as the feedstock for supplying the reducing organic substance. With regard to the kind of plant to be used for stem and leaf squeezing, stems and leaves of the plant bodies described in the foregoing paragraphs may be preferably used. In particular, a plant rich in total polyphenols is preferred from the viewpoint of potency. Further, from the viewpoint of the cost of the raw material, squeezed juices of, for example, field horsetail, Japanese cypress, pine, and Japanese cedar are preferably used.

### (c) Plant Dry Distillation Liquid

The 'plant dry distillation liquid' is preferably used as the feedstock for supplying the reducing organic substance. The feedstock is considered to include a large amount of a polyphenol and to include many molecules of reducing organic substances such as phenols, organic acids, carbonyls, alcohols, amines, basic components, and other neutral components.

Herein, the plant dry distillation liquid refers to a dry distillation liquid obtained by thermal degradation of a plant body in a reducing condition (sticky and brown liquid). The liquid has a red-brown to dark brown color in appearance. A stock solution may be used without additional treatments, but a concentrate, a diluted solution, or a dried product thereof may be used.

Specific examples of the plant dry distillation liquid may include wood vinegar, bamboo vinegar, and chaff vinegar. From the viewpoint of the cost of the raw material, those may be preferably used.

### (d) Roasted Coffee Beans

A raw material derived from the 'roasted coffee beans' is preferably used as the feedstock for supplying the reducing organic substance. The feedstock is very rich in polyphenols.

In the present invention, the roasted coffee beans may be used without additional treatments or after pulverization. Further, a component extracted from the pulverized product with water or hot water (so-called brewed coffee component) may be used. Further, a residue obtained after extraction with water or hot water (so-called coffee grounds) may be used.

In particular, from the viewpoint of the cost of the raw material, the 'coffee grounds,' which are disposed of in a large amount after extraction of a coffee component, are most preferably used.

Herein, the roasted coffee beans may include any product as long as the product is obtained by roasting coffee beans according to a usual method. So-called milled (ground) coffee beans are also included in the roasted coffee beans. Further, coffee beans which are roasted after grinding may be used.

In this case, any seeds of *Coffea* such as *Coffea Arabica* (Arabica), *C. canephora* (Robusta), or *C*. *liberica* (Liberica coffee) may be used as the coffee beans. It should be noted that raw coffee beans may be used, and coffee beans dried and preserved as usual are preferably used. From the viewpoint of the cost of the raw material, off-specification coffee beans are industrially preferably used.

In this case, roastingmaybe carried out by any of usual methods. Examples thereof may include open fire roasting, hot air roasting, far-infrared roasting, microwave roasting, heated steam roasting, and low temperature roasting.

Further, grinding only needs to be carried out so that usual ground coffee beans are obtained by using a coffee mill, a grinder, a stone mill, or the like, and the level of grinding widely ranges from coarsely grinding to grinding into powder. From the viewpoint of the reaction efficiency with iron, surface areas of the coffee beans are preferably increased, and hence the coffee beans are preferably subjected to, for example, fracture, pulverization, or powderization.

### (d) Tea Leaves

A raw material derived from the 'tea leaves' is preferably used as the feedstock for supplying the reducing organic substance. The raw material is very rich in polyphenols.

In the present invention, the tea leaves may be used without additional treatments or after pulverization. Further, a component extracted from the pulverized tea leaves with water or hot water (so-called brewed tea component) may be used. Further, a residue obtained after extraction with water or hot water (so-called tea dregs) may be used. In particular, from the viewpoint of the cost of the raw material, the 'tea dregs,' which are disposed of in a large amount after extraction of a tea component, are most preferably used.

In this case, any picked stems and leaves of *Camellia sinensis,* which is one of the tea plants, may be used as the tea leaves. Further, a picking method may be any one, but from the viewpoint of the cost, mechanical picking is particularly preferred.

It should be noted that, in the picked tea leaves, cell contents are mixed to cause oxidative fermentation, but tea leaves at any stage of fermentation may be used in the present invention. For example, there may be used: green tea obtained by suppressing oxidative fermentation by heating (such as green tea of middle grade, coarse green tea, twig tea, or roasted green tea) ; blue tea obtained by fermenting the leaves to some degree (such as oolong tea) ; black tea obtained by completely fermenting the leaves; and dark tea obtained by oxidative fermentation and fermentation with aspergilli (such as pu'er tea). Preferred examples thereof may include green tea, black tea, and oolong tea. It should be noted that, from the viewpoint of the cost of the raw material, off-specification tea leaves are industrially preferably used.

Further, from the viewpoint of the reaction efficiency with iron, surface areas of the tea leaves are preferably increased, and hence the tea leaves are preferably subjected to, for example, fracture, pulverization, or powderization before use.

### [Iron-supplying Source]

In the present invention, any of a source for supplying divalent iron, a source for supplying trivalent iron, and a source for supplying metallic iron may be used as the iron element-supplying source. Further, a mixture of a plurality of the sources may be used.

Herein, examples of the source for supplying divalent iron may include: water-soluble iron compounds, such as iron(II) chloride, iron(II) nitrate, iron(II) sulfate, iron(II) hydroxide, iron(II) oxide, iron(II) acetate, iron(II) lactate, iron (II) sodium citrate, andiron(II) gluconate; and insoluble iron compounds, such as iron(II) carbonate and iron(II) fumarate.

It should be noted that, of the divalent iron compounds, even a compound insoluble in water may be directly used as the iron-supplying source of the present invention because the compound becomes soluble in water by the chelating ability of the reducing organic substance.

In addition, examples of the source for supplying trivalent iron may include: water-soluble iron compounds, such as iron (III) chloride, iron (III) sulfate, iron(III) citrate, iron (III) ammonium citrate, and EDTA iron (III) ; and insoluble iron compounds, such as iron(III) oxide, iron(III) nitrate, iron(III) hydroxide, and iron(III) pyrophosphate.

In addition, as a natural source containing a large amount of a trivalent iron compound, there may be given: soils, such as Akadama soil, Kanuma soil, loam (allophanic iron-rich soil), laterite (iron(III) oxide-rich soil), and goethite (soil containing an amorphous mineral) ; natural iron ores, such as pyrite, marcasite, siderite, magnetite, and goethite; iron sand in the form of sand dust obtained from any of the iron ores; and a biogenic substance, such as heme iron or a seashell. It should be noted that many of trivalent iron compounds contained in soils or iron ores are usually insoluble in water.

Examples of the source for supplying metallic iron may include iron materials, such as smelted iron and alloys. Further, rust may be used as the source. It should be noted that the metallic iron is usually insoluble in water.

Further, an aqueous solution containing divalent iron ion and/or trivalent iron ion, prepared by dissolving any of the above-mentioned iron compounds in water, may be used.

It should be noted that, of the above-mentioned iron-supplying sources, even a source insoluble in water may be directly used as the iron-supplying source of the present invention because the iron becomes soluble in water by the chelating ability of the reducing organic substance.

Of those, a water-soluble iron compound is preferably used to efficiently produce the photocatalyst of the present invention. In particular, for example, inexpensive iron chloride or iron sulfate is preferably used. It should be noted that the iron in the compound to be used may be divalent or trivalent.

Further, to produce the photocatalyst in light of the cost of the raw material and stable supply, a natural product of soil (in particular, Akadama soil, Kanuma soil, loam, or the like) or metallic iron is preferably used as the iron-supplying source.

### [Mixing Treatment]

In the present invention, the reducing organic substance (or the feedstock for supplying the reducing organic substance) is mixed with the iron-supplying source (or iron ion) in the presence of water to convert iron into divalent iron ion, and thus a reaction product that coordinates the divalent iron ion and is an active component having photocatalytic ability can be obtained.

### ·Mixing Ratio of Raw Materials

The raw materials may be mixed in the following ratio. The iron-supplying source may be mixed so that the content of the iron-supplying source is 0.1 part by weight or more, preferably 0.5 part by weight or more, more preferably 1 part by weight or more, still more preferably 2 parts by weight or more, particularly preferably 3 parts by weight or more, even more preferably 4 parts by weight or more, in terms of weight of iron element, with respect to 100 parts by weight, in terms of dry weight, of the reducing organic substance or the feedstock for supplying the reducing organic substance. When the ratio of the iron element is too low (if the mixing ratio of the reducing organic substance to the iron element is too high), an excessive amount of the reducing organic substance may act as a radical-scavenging substance (scavenger) to inhibit the photocatalytic activity.

Further, the upper limit of the amount of the iron element may be, for example, 10 parts by weight or less, preferably 8 parts by weight or less, more preferably 6 parts by weight or less in terms of weight of the iron element. When the ratio of the iron element is too high (if the mixing ratio of the reducing organic substance to the iron element is too low), divalent iron ion cannot be maintained to deteriorate the photocatalytic activity, which is not preferred.

### ·Mixing Procedure

The mixing procedure of the present invention is performed in the presence of water. In this case, the expression "in the presence of water" refers to a condition where the reducing organic substance can react with iron using water as a medium. Specifically, the reaction is considered as a reaction in which the reducing organic substance converts ferric iron ion into a reduced iron state (a state of divalent iron ion, Fe²⁺) to form a complex.

The amount of water may be a liquid amount in which the raw materials can be at least mixed and stirred, or may be such an amount that a mixture of the raw materials (reducing organic substance and iron) becomes wet.

It should be noted that the water to be used may be any water as long as the reaction occurs, and examples thereof may include tap water, well water, underground water, river water, deionized water, and distilled water.

It should be noted that, when the squeezed plant juice, the plant dry distillation liquid, or the like is used in a liquid state as the feedstock for supplying the reducing organic substance, the juice, the liquid, or the like may be mixed directly with the iron-supplying source for the reaction without addition of another medium.

With regard to the mixing procedure, mixing may be carried out simply by stirring, but may be carried out with a mixer, a large-scale stirring vessel, a Vortex mixer, a shaker, or the like.

In this case, the temperature of the watermaybe any temperature as long as the water is in a liquid state (for example, at 1 atm, from 1°C to 100°C).

The temperature may be about room temperature (for example, from 10°C to 35°C) at which no heating is required. When the mixing is carried out with heating, the heating is suitably carried out at 40°C or more, preferably 50°C or more to promote production of the reaction product. The upper limit of the temperature may be, for example, 200°C (in the case of heating under increased pressure), but from the viewpoint of production cost, the temperature is desirably 100°C or less, which is the boiling point of water in usual heating under a normal pressure condition, preferably 90°C or less, more preferably 70°C or less. It should be noted that, in order to suppress thermal degradation of the reducing organic substance under a reaction condition of 100°C or more, it is more suitable to carry out mixing in a sealed container.

With regard to a mixing time, mixing only needs to be carried out for about 10 seconds or more until the reducing organic substance is brought sufficiently into contact with iron, but in order to improve uniformity, it is desirable to carry out mixing treatment for preferably 1 minute or more, more preferably 3 minutes or more, still more preferably 5 minutes or more.

In addition, with regard to the upper limit, in order to prevent putrescence of the organic substance due to propagation of microorganisms, mixing is desirably carried out within 10 days, preferably 7 days, more preferably 5 days, still more preferably 3 days, particularly preferably 1 day. However, when sterilization treatment is carried out, the upper limit is not particularly specified.

It should be noted that, when an insoluble iron compound is used as the iron-supplying source, the reaction time after mixing may be extended to increase the amount of the reaction product.

### [Photocatalyst]

The reaction product (reaction product of the reducing organic substance and iron) obtained through the above-mentioned process has an excellent photocatalytic activity. In the reaction product, the reducing organic substance is considered to convert ferric iron ion into a divalent iron state (Fe²⁺ state) to form a complex.

The reaction product obtained through the above-mentioned process may be used as the photocatalyst without additional treatments as a supernatant or a precipitate in a water-containing state obtained after the reaction. In addition, the supernatant or precipitate may be separated, collected, and used as the photocatalyst. In addition, a driedproduct (forexample, natural drying or roasting) of the supernatant and/or precipitate, or a supernatant or suspension obtained by further dissolving the dried product in water may be used as the photocatalyst.

### · Characteristics of Photocatalyst of the Present Invention

Even when the reaction product is irradiated with light having a wide range of wavelengths of from 200 nm to 1,400 nm, that is, even when the reaction product is irradiated with not only ultraviolet light but also visible light or infrared light, the reaction product has a property of absorbing the light to exhibit an excellent photocatalytic activity.

The term 'ultraviolet light' as used herein refers to light in a wavelength range of 380 nm or less. Further, the term 'visible light' refers to light in a wavelength range of from 380 nm to 750 nm, which is visible for human eyes. The visible light specifically refers to light in a wavelength range of from 380 nm to 450 nm (purple light), from 450 nm to 495 nm (blue light), from 495 nm to 570 nm (green light), from 570 nm to 590 nm (yellow light), from 590 nm to 620 nm (orange light), or from 620 nm to 750 nm (red light). In addition, the infrared light refers to light in a wavelength range of 750 nm or more.

When the reaction product is irradiated with ultraviolet light, the product exhibits a very strong photocatalytic activity. In particular, when the reaction product is irradiated with near-ultraviolet light having a wavelength of from 200 nm to 380 nm, the intensity of the activity shows the potency much higher than that of titanium oxide.

Further, even when the reaction product is irradiated with visible light and infrared light each in a wavelength range in which titanium oxide does not exhibit the activity, the product exhibits a strong photocatalytic activity. In the case of visible light, the reaction product exhibits a strong activity by irradiation with light having a particularly short wavelength, i.e., in a wavelength range of from purple light to blue light (from 380 nm to 495 nm). In the case of infrared light, the reaction product exhibits a strong activity by irradiation with near-infrared light in a wavelength range of from 750 nm to 1,400 nm (particularly from about 900 nm to about 1,300 nm, more particularly from about 1,100 nm to about 1,300 nm). Such photocatalytic activity provided by visible light or infrared light is not found in conventional technologies.

The reaction product absorbs energy of light irradiated to exhibit an activity to degrade, for example, organic substances present in the vicinity of the product. The activity is considered as a phenomenon caused by radicals generated by the photocatalyst excited by the energy of light.

When the reaction product is continuously irradiated with light, the product continuously exhibits a photocatalytic activity during irradiation. Further, even when the light irradiation is suspended, the product exhibits the photocatalytic activity by being irradiated again. That is, the reaction product is a material that can be used repeatedly as a photocatalyst.

This is probably because a resonant structure in a molecule of the reaction product (Fe²⁺ complex of the reducing organic substance) transmits the light energy to Fe²⁺ to efficiently generate radicals, and the molecule has a stable structure that scavenges the radicals by the resonant structure even when attacked by the radicals.

### [Specific Use Applications of Photocatalyst]

The photocatalyst (reaction product of the reducing organic substance and iron) of the present invention is a highly safe substance for a human body and an environment, and hence can be used in various applications, such as medicine, food, public health, agriculture, and industry.

For example, when ascorbic acid or the polyphenol is used as the reducing organic substance, the photocatalyst is particularly expected to be used in the food field because ascorbic acid or the polyphenol is a substance derived from a supplying feedstock derived from food. Ascorbic acid is particularly suitable because the ascorbic acid is colorless and transparent.

In addition, when the plant dry distillation liquid is used as the feedstock for supplying the reducing organic substance, the component contains a substance having a slight odor. However, the feedstock is very inexpensive, and hence is expected to be used in the fields of, for example, agriculture, medicine, and public health.

### · Degradation of Organic Substance

The present invention can be used for degradation of general organic substances based on the organic substance degradation activity of the photocatalyst, and can be preferably used particularly for degradation of organic pollutants and harmful substances. That is, the present invention can be used usefully in one step of environmental cleanup.

The terms "pollutants" and "harmful substances" as used herein refer to substances that cause water pollution, soil pollution, or air pollution. Examples of the substances may include organic substances that are contained in domestic sewage, human excrement water, factory effluent, polluted river water and lake water, soil of dump sites, industrial waste, agricultural land, and old factory site and that are harmful to a human body and an environment.

Specific examples of the organic substance to be degraded may include organic wastes, such as detergents, food and beverage residues, human excrement, feces, agrichemicals, malodorous substances, waste oils, dioxin, PCB, DNA, RNA, and proteins.

When the photocatalyst of the present invention is used as an active component of an organic substance decomposer, as the form of the organic substance decomposer, for example, there may be given: solid forms, such as powder, granule, sheet, board, cube, and sponge forms; and liquid forms, such as a concentrate and a liquid ampule. Examples thereof may further include a powdery form, a form of a solid mixed with an excipient or the like, a form of filling in a capsule, and a form of gel.

In the present invention, when the photocatalyst is, for example, sprayed, dispersed, added, mixed, applied, or kneaded with respect to an object to be degraded, and then irradiated with light, the photocatalyst can degrade organic substances.

For the amount of the photocatalyst to be used, the photocatalyst may be used as a solution having such a concentration that the photocatalyst can exhibit an organic substance degradation action. For example, it is desirable that the photocatalyst be used by being prepared into a solution having a concentration in terms of iron of 0.001 ppm or more, preferably 0.01 ppm or more, more preferably 0.05 ppm or more, still more preferably 0.1 ppm or more, particularly preferably 0.5 ppm or more, even more preferably 1 ppm or more, still even more preferably 2.5 ppm or more, yet still even more preferably 5 ppm or more, particularly even more preferably 5.5 ppm or more, still even more preferably 10 ppm or more, particularly still even more preferably 20 ppm or more.

Further, the upper limit of the concentration is not particularly specified, and may be, for example, 40, 000 ppm or less, preferably 10,000 ppm or less, more preferably 5,000 ppm or less, still more preferably 1,000 ppm or less, particularly preferably 750 ppm, even more preferably 500 ppm or less, in terms of iron.

The photocatalyst has a very strong degradation effect and hence can degrade persistent organic substances (for example, basic fuchsin) efficiently. For example, when the photocatalyst is irradiated with light of 100 W/m², the photocatalyst can degrade organic substances in an amount of at least 2.5 mg/L or more per dray, at most 35 mg/L or more per day.

### · Sterilization

The photocatalyst of the present invention can be used for sterilization in various fields based on its strong organic substance degradation action. Specific examples of the obj ect to be sterilized may include medical equipment, walls of hospital rooms, affected areas of patients, clothes, bedclothes, lines of food manufacturing equipment, food materials, kitchen goods such as a cutting board and a kitchen knife, dishes, toilet seats, handrails, farm equipment, nutriculture devices, and nutrient solutions. Unlike a usual sterilization method using titanium oxide, the photocatalyst of the present invention can be used by being irradiated with visible light and infrared light, and hence applications and scenes of use of the photocatalyst are significantly enlarged.

Further, the object to be sterilized may include not only bacteria but also eukaryotic microorganisms, algae, archaea, viruses, and viroids.

When the photocatalyst of the present invention is used as an active component of a sanitizer, as the form of the sanitizer, for example, there may be given: solid forms, such as powder, granule, sheet, board, cube, and sponge forms; and liquid forms, such as a diluted solution, a concentrate, and a liquid ampule. Examples thereof may further include a powdery form, a form of a solid mixed with an excipient or the like, a form of filling in a capsule, and a form of gel.

In the present invention, when the photocatalyst is, for example, sprayed, dispersed, added, mixed, applied, or kneaded with respect to an object to be degraded, and then irradiated with light, sterilization can be performed.

For the amount of the photocatalyst to be used, the photocatalyst may be used as a solution having such a concentration that the photocatalyst can exhibit a sterilization effect. For example, it is desirable that the photocatalyst be used by being prepared into a solution having a concentration in terms of iron of 0.001 ppm or more, preferably 0.01 ppm or more, more preferably 0.05 ppm or more, still more preferably 0.1 ppm or more, particularly preferably 0.5 ppm or more, even more preferably 1 ppm or more, still even more preferably 2.5 ppm or more, yet still even more preferably 5 ppm or more, particularly even more preferably 5.5 ppm or more, still even more preferably 10 ppm or more, particularly still even more preferably 20 ppm or more.

Further, the upper limit of the concentration is not particularly specified, and may be, for example, 40, 000 ppm or less, preferably 10,000 ppm or less, more preferably 5,000 ppm or less, still more preferably 1,000 ppm or less, particularly preferably 750 ppm, even more preferably 500 ppm or less, in terms of iron.

The photocatalyst has a very strong sterilization effect. Accordingly, in the case of, for example, surface sterilization, the photocatalyst can exhibit a sufficient sterilization effect when irradiated with sunlight for about several minutes, preferably for 10 minutes or more, more preferably for 20 minutes or more.

Further, even in the case of irradiation with relatively weak light, such as LED or fluorescent light, the photocatalyst can exhibit a sufficient sterilization effect by performing treatment for 1 hour or more, preferably for 6 hours or more, more preferably for 12 hours or more.

### Examples

The present invention hereinafter is specifically described by way of Examples, but the scope of the present invention is not limited by Examples.

### [Example 1] "Raw Material Containing Reducing Organic Substance"

Various raw materials were investigated for the presence or absence of an activity to reduce trivalent iron into divalent iron so as to judge whether the materials were reducing organic substances or not.

### (1) 'Investigation on Iron-reducing Ability'

Respective aqueous solutions (solutions containing 0.1 wt% raw materials and 0.1 wt% iron chloride) were prepared so that the solutions contained iron (III) chloride in the same weight in terms of iron element with respect to 100 parts by weight (in terms of dry weight) of each of raw materials shown in Table 1 and allowed to stand still at room temperature for several minutes. After that, dipyridyl (2 g/L) and acetic acid (100 g/L) were added to and mixed in each of the aqueous solutions to examine the presence or absence of a color reaction. In this connection, dipyridyl is a substance which does not react with trivalent iron and remains colorless, but turns red when reacted with divalent iron. Dipyridyl is used for detection of divalent iron. Further, as a control, an aqueous solution of 0.1 wt% iron(III) chloride was prepared and subjected to the same procedure. The results are shown in Table 1.

As a result, solutions containing various raw materials derived from plant bodies (Samples 1-1 to 1-8), solutions containing polyphenols (Samples 1-9 to 1-13), and a solution containing ascorbic acid (Sample 1-14) turned red. That is, the raw materials were found to contain reducing organic substances having actions of reducing trivalent iron into divalent iron. Further, the divalent iron reduced in this procedure was found to be maintained in a divalent iron state stably.

On the other hand, a solution containing citric acid (Sample 1-15) remained colorless. That is, the raw material was found to have no action of reducing trivalent iron into divalent iron.

### (2) Discussion

The results revealed that the polyphenols and ascorbic acid were reducing organic substances having iron-reducing ability. The results further revealed that the various raw materials derived from the plant bodies (in particular, polyphenol-rich raw materials) served as feedstocks for supplying reducing organic substances having iron-reducing ability. Further, the divalent iron ion was maintained stably, and hence the reducing organic substances were considered to form complex structures of divalent iron ion.

On the other hand, citric acid is a substance known as a chelating agent having reducing ability, but was found to have no ability to reduce iron.

**[Table 1]**

| | Raw material | Reaction product | Red color development |
|---|---|---|---|
| Sample 1-1 | Tea dregs | Tea leaf component/iron | + |
| Sample 1-2 | Coffee grounds | Roasted coffee bean component/iron | + |
| Sample 1-3 | Squeezed grape juice | Squeezed grape juice component/iron | + |
| Sample 1-4 | Chaff vinegar | Chaff vinegar component/iron | + |
| Sample 1-5 | Squeezed red cabbage juice | Squeezed red cabbage juice component/iron | + |
| Sample 1-6 | Squeezed banana juice | Squeezed banana juice component/iron | + |
| Sample 1-7 | Cacao powder | Cacao powder component/iron | + |
| Sample 1-8 | Turmeric powder | Turmeric powder component/iron | + |
| Sample 1-9 | Grape polyphenol | Grape polyphenol/iron | + |
| Sample 1-10 | Caffeic acid | Caffeic acid/iron | + |
| Sample 1-11 | Chlorogenic acid | Chlorogenic acid/iron | + |
| Sample 1-12 | Tannic acid | Tannic acid/iron | + |
| Sample 1-13 | Catechin | Catechin/iron | + |
| Sample 1-14 | Ascorbic acid | Ascorbic acid/iron | + |
| Sample 1-15 | Citric acid | Citric acid/iron | - |
| Control | None | | - |

### [Example 2] "Wavelength to Excite Photocatalytic Activity"

'Reaction products of reducing organic substances and iron' were prepared using tea dregs or coffee grounds as raw materials to examine photocatalytic activities of the substances.

### (1) 'Measurement of Photocatalytic Activity'

Iron (III) chloride (FeCl₃) was mixed in an amount of 4 parts by weight in terms of iron element with respect to 100 parts by weight (in terms of dry weight) of tea dregs (residue of extraction of tea leaves with hot water) or coffee grounds (residue of extraction of roasted and ground coffee beans with hot water), and water was added thereto in twice the total weight of the mixture, followed by thermal treatment at 98°C for 1 hour. Thus, a reaction product was obtained. A filtrate obtained by filtration was referred to as "tea leaf component/iron" (Sample 2-1) or "roasted coffee bean component/iron" (Sample 2-2). Further, as a comparative sample, titanium oxide (TiO₂: anatase-type titanium(IV) oxide, particle size: 100 nmto 300 nm, manufactured by Wako Pure Chemical Industries, Ltd.) (Comparison 2-1) was prepared.

Subsequently, a plurality of aqueous solutions of 3.5 ppm basic fuchsin were prepared for each of the samples so that the solutions each contained any one of the samples or the comparative sample at a concentration of 35 ppm in terms of iron element (for titanium oxide,in termsof titanium element). The aqueous solutions were allowed to stand still and irradiated with LED with different wavelengths for 24 hours while basic fuchsin was quantified with time. The irradiation intensities of the LED in this procedure are as follows. The irradiation intensity of ultraviolet light (375 nm) was 1mW/cm². The irradiation intensities of visible light (blue light (470 nm), green light (525 nm), yellow light (570 nm), red light (660 nm)) and infrared light (940 nm, 1,200 nm) were each 100 nmol/m²/sec in terms of photon density. Further, the basic fuchsin was quantified by measuring absorbances at 540 nm. It should be noted that, as a control, the aqueous solutions were allowed to stand still for 24 hours under dark conditions while basic fuchsin was quantified with time.

Absorbances at 540 nm (results of quantification of basic fuchsin) were measured, and degradation rates were calculated and are shown in Tables 2-A to 2-D.

The results revealed that, in the solutions containing "tea leaf component/iron" (Sample 2-1) or "roasted coffee bean component/iron" (Sample 2-2), basic fuchsin (persistent organic substance) was degraded by irradiation not only with ultraviolet light (375 nm) but also with visible light (470 nm, 525 nm, 570 nm, and 660 nm) and infrared light (940 nm and 1,200 nm).

Specifically, the solution containing "tea leaf component/iron" (Sample 2-1) was found to exhibit a high degradation activity when irradiated with visible light and infrared light (in particular, infrared light having a longer wavelength of 1,200 nm). In particular, when the solution was irradiated with ultraviolet light, rapid degradation was observed in a short time (within 6 hours or less after the start of irradiation).

Further, the solution containing "roasted coffee bean component/iron" (Sample 2-2) was found to exhibit a degradation activity when irradiated with visible light. In particular, the solution was found to exhibit a high activity when irradiated with infrared light (in particular, infrared light having a longer wavelength of 1,200 nm). In particular, when the solution was irradiated with ultraviolet light, very rapid degradation was observed in a short time (within 6 hours or less after the start of irradiation).

On the other hand, in the solution containing titanium oxide (Comparison 2-1) prepared as a comparative sample, basic fuchsin was observed to be degraded only by irradiation with ultraviolet light having a wavelength of 375 nm. It should be noted that no degradation of basic fuchsin was observed at wavelengths larger than those of visible light (>470 nm). That is, the photocatalytic activity of titanium oxide was detected only by irradiation with ultraviolet light.

### (2) 'Discussion'

The results revealed that the reaction products of the reducing organic substances prepared using the tea dregs or coffee grounds as raw materials and iron had strong photocatalytic ability. In particular, the reaction products were found to exhibit strong photocatalytic activities even when irradiated with visible light and infrared light having wavelengths at which titanium oxide (related-art photocatalyst) did not react.

The reaction products were found to exhibit particularly strong photocatalytic activities when irradiated with ultraviolet light and infrared light (in particular, infrared light having a longer wavelength). In particular, in the case of irradiation with ultraviolet light, the reaction products were found to exhibit rapid and strong photocatalytic activities in a short time as compared to titanium oxide.

**[Table 2-A]**

| | Reaction product or compound | Rate of degradation by irradiation at different wavelengths (nm) (6 hr later) | | | | | | |
|---|---|---|---|---|---|---|---|---|
| | | 375 nm | 470 nm | 525 nm | 570 nm | 660 nm | 940 nm | 1,200 nm |
| Sample 2-1 | Tea leaf component/iron | 78 | 50 | 26 | 17 | 15 | 36 | 31 |
| Sample 2-2 | Roasted coffee bean component/iron | 97 | 27 | 17 | 9 | 12 | 35 | 24 |
| Comparison 2-1 | Titanium oxide | 38 | 0 | 0 | 0 | 0 | 0 | 0 |

**[Table 2-B]**

| | Reaction product or compound | Rate of degradation by irradiation at different wavelengths (nm) (12 hr later) | | | | | | |
|---|---|---|---|---|---|---|---|---|
| | | 375 nm | 470 nm | 525 nm | 570 nm | 660 nm | 940 nm | 1,200 nm |
| Sample 2-1 | Tea leaf component/iron | 86 | 56 | 43 | 30 | 27 | 52 | 50 |
| Sample 2-2 | Roasted coffee bean component/iron | 100 | 36 | 27 | 13 | 19 | 44 | 35 |
| Comparison 2-1 | Titanium oxide | 59 | 0 | 0 | 0 | 0 | 0 | 0 |

**[Table 2-C]**

| | Reaction product or compound | Rate of degradation by irradiation at different wavelengths (nm) (18 hr later) | | | | | | |
|---|---|---|---|---|---|---|---|---|
| | | 375 nm | 470 nm | 525 nm | 570 nm | 660 nm | 940 nm | 1,200 nm |
| Sample 2-1 | Tea leaf component/iron | 90 | 67 | 54 | 39 | 47 | 66 | 66 |
| Sample 2-2 | Roasted coffee bean component/iron | 100 | 43 | 33 | 24 | 30 | 37 | 37 |
| Comparison 2-1 | Titanium oxide | 76 | 0 | 0 | 0 | 0 | 0 | 0 |

**[Table 2-D]**

| | Reaction product or compound | Rate of degradation by irradiation at different wavelengths (nm) (24 hr later) | | | | | | |
|---|---|---|---|---|---|---|---|---|
| | | 375 nm | 470 nm | 525 nm | 570 nm | 660 nm | 940 nm | 1,200 nm |
| Sample 2-1 | Tea leaf component/iron | 91 | 72 | 61 | 71 | 55 | 80 | 86 |
| Sample 2-2 | Roasted coffee bean component/iron | 100 | 49 | 37 | 43 | 34 | 55 | 56 |
| Comparison 2-1 | Titanium oxide | 88 | 0 | 0 | 0 | 0 | 0 | 0 |

### [Example 3] "Investigation Using Various Reducing Organic Substances"

Reaction products of various reducing organic substances and iron were prepared and it was investigated whether the products had photocatalytic activities.

### (1) 'Measurement of Photocatalytic Activity'

Iron (III) chloride (FeCl₃) was mixed in an amount of 4 parts by weight in terms of iron element with respect to 100 parts by weight (in terms of dry weight) of ascorbic acid, grape polyphenol, catechin, chlorogenic acid, caffeic acid, tannic acid, or chaff vinegar, and water was added thereto in twice the total weight of the mixture, followed by thermal treatment at 98°C for 1 hour. Thus, reaction products shown in Table 3 were obtained (Samples 3-1 to 3-7). Further, as a control sample, titanium oxide (TiO₂: anatase-type titanium(IV) oxide, particle size: 100 nm to 300 nm, manufactured by Wako Pure Chemical Industries, Ltd.) was prepared (Comparison 3-1).

Subsequently, a plurality of aqueous 3.5 ppm basic fuchsin solutions were prepared so that the solutions each contained any one of the samples or the comparative sample at a concentration of 5.5 ppm in terms of iron element (for titanium oxide, in terms of titanium element). The aqueous solutions were allowed to stand still and irradiated with LED with different wavelengths for 24 hours while basic fuchsin was quantified with time. Quantification of basic fuchsin and irradiation with LED were carried out in the same manner as in Example 2. Further, as a control, the aqueous solutions were allowed to stand still for 24 hours under dark conditions while basic fuchsin was quantified with time.

Absorbances at 540 nm (results of quantification of basic fuchsin) were measured, and degradation rates were calculated and are shown in Tables 3-A to 3-D.

The results revealed that, in the solutions containing the reaction products of the various reducing organic substances and iron, basic fuchsin was degraded by irradiation with light having any of the wavelengths of ultraviolet light (375 nm), visible light (470 nm, 525 nm, 570 nm, and 660 nm), and infrared light (940 nm and 1,200 nm). That is, when the solutions were irradiated with visible light and infrared light (in particular, infrared light having a longer wavelength of 1, 200 nm), high degradation activities were detected. Further, when the solutions were irradiated with ultraviolet light, rapid degradation activities were detected in a short time.

In particular, the reaction products of the polyphenols, such as catechin, chlorogenic acid, caffeic acid, and tannic acid, and iron were found to exhibit strong photocatalytic activities when irradiated with visible light and infrared light (Samples 3-3 to 3-6). Further, all of the reaction product samples were found to exhibit particularly strong photocatalytic activities when irradiated with ultraviolet light (Samples 3-1 to 3-7).

On the other hand, the solution containing titanium oxide prepared as a comparative sample was found to exhibit no degradation activity when irradiated with visible light and infrared light, and found to exhibit a degradation activity only when irradiated with ultraviolet light (Comparison 3-1). Further, the degradation was performed in a linear manner at a slow rate.

### (2) 'Discussion'

From the results, the photocatalytic activity was considered to be a property common in the reaction products of the reducing organic substances having iron-reducing ability and iron. In particular, the results revealed that the reaction products exhibited the photocatalytic activities when irradiated with visible light and infrared light having wavelengths at which titanium oxide (related-art photocatalyst) did not react.

The results further revealed that the photocatalytic activities provided by irradiation with visible light and infrared light were properties highly retained by the reaction products of the present invention.

**[Table 3-A]**

| Reaction product or compound | | Rate of degradation by irradiation at different wavelengths (nm) (6hrlater) | | | | | | |
|---|---|---|---|---|---|---|---|---|
| | | 375 nm | 470 nm | 525 nm | 570 nm | 660 nm | 940 nm | 1,200 nm |
| Sample 3-1 | Ascorbic acid/iron | 50 | 21 | 28 | 30 | 8 | 25 | 8 |
| Sample 3-2 | Grape polyphenol/iron | 97 | 26 | 26 | 32 | 19 | 25 | 20 |
| Sample 3-3 | Catechin/iron | 76 | 23 | 35 | 40 | 42 | 28 | 42 |
| Sample 3-4 | Chlorogenic acid/iron | 60 | 33 | 40 | 33 | 46 | 40 | 46 |
| Sample 3-5 | Caffeic acid/iron | 70 | 37 | 45 | 45 | 62 | 41 | 61 |
| Sample 3-6 | Tannic acid/iron | 70 | 36 | 50 | 46 | 60 | 35 | 60 |
| Sample 3-7 | Chaff vinegar component/iron | 70 | 42 | 30 | 30 | 26 | 28 | 26 |
| Comparison 3-1 | Titanium oxide (TiO₂) | 18 | 0 | 0 | 0 | 0 | 0 | 0 |
| Control | None | 8 | 0 | 0 | 0 | 0 | 0 | 0 |

**[Table 3-B]**

| Reaction product or compound | | Rate of degradation by irradiation at different wavelengths (nm) (12 hr later) | | | | | | |
|---|---|---|---|---|---|---|---|---|
| | | 375 nm | 470 nm | 525 nm | 570 nm | 660 nm | 940 nm | 1,200 nm |
| Sample 3-1 | Ascorbic acid/iron | 97 | 26 | 50 | 34 | 21 | 27 | 21 |
| Sample 3-2 | Grape polyphenol/iron | 99 | 35 | 30 | 33 | 30 | 29 | 27 |
| Sample 3-3 | Catechin/iron | 92 | 35 | 43 | 44 | 52 | 40 | 52 |
| Sample 3-4 | Chlorogenic acid/iron | 73 | 45 | 50 | 43 | 60 | 47 | 59 |
| Sample 3-5 | Caffeic acid/iron | 84 | 50 | 56 | 54 | 72 | 48 | 72 |
| Sample 3-6 | Tannic acid/iron | 85 | 60 | 57 | 56 | 71 | 42 | 71 |
| Sample 3-7 | Chaff vinegar component/iron | 88 | 47 | 33 | 33 | 34 | 32 | 34 |
| Comparison 3-1 | Titanium oxide (TiO₂) | 32 | 0 | 0 | 0 | 0 | 0 | 0 |
| Control | None | 11 | 0 | 0 | 0 | 0 | 0 | 0 |

**[Table 3-C]**

| Reaction product or compound | | Rate of degradation by irradiation at different wavelengths (nm) (18 hr later) | | | | | | |
|---|---|---|---|---|---|---|---|---|
| | | 375 nm | 470 nm | 525 nm | 570 nm | 660 nm | 940 nm | 1,200 nm |
| Sample 3-1 | Ascorbic acid/iron | 99 | 91 | 61 | 44 | 34 | 31 | 33 |
| Sample 3-2 | Grape polyphenol/iron | 99 | 45 | 60 | 46 | 43 | 33 | 43 |
| Sample 3-3 | Catechin/iron | 97 | 42 | 50 | 56 | 61 | 45 | 61 |
| Sample 3-4 | Chlorogenic acid/iron | 82 | 53 | 60 | 53 | 70 | 52 | 68 |
| Sample 3-5 | Caffeic acid/iron | 86 | 55 | 64 | 64 | 74 | 53 | 79 |
| Sample 3-6 | Tannic acid/iron | 92 | 70 | 67 | 63 | 75 | 51 | 75 |
| Sample 3-7 | Chaff vinegar component/iron | 96 | 52 | 40 | 40 | 50 | 36 | 45 |
| Comparison 3-1 | Titanium oxide (TiO₂) | 46 | 0 | 0 | 0 | 0 | 0 | 0 |
| Control | None | 16 | 0 | 0 | 0 | 0 | 0 | 0 |

**[Table 3-D]**

| Reaction product or compound | | Rate of degradation by irradiation at different wavelengths (nm) (24 hr later) | | | | | | |
|---|---|---|---|---|---|---|---|---|
| | | 375 nm | 470 nm | 525 nm | 570 nm | 660 nm | 940 nm | 1,200 nm |
| Sample 3-1 | Ascorbic acid/iron | 100 | 98 | 70 | 60 | 57 | 35 | 34 |
| Sample 3-2 | Grape polyphenol/iron | 100 | 53 | 65 | 53 | 70 | 36 | 43 |
| Sample 3-3 | Catechin/iron | 100 | 50 | 55 | 63 | 65 | 51 | 61 |
| Sample 3-4 | Chlorogenic acid/iron | 85 | 60 | 65 | 62 | 73 | 58 | 68 |
| Sample 3-5 | Caffeic acid/iron | 90 | 62 | 70 | 70 | 80 | 58 | 80 |
| Sample 3-6 | Tannic acid/iron | 95 | 75 | 71 | 70 | 81 | 57 | 75 |
| Sample 3-7 | Chaff vinegar component/iron | 99 | 57 | 42 | 47 | 64 | 39 | 45 |
| Comparison 3-1 | Titanium oxide (TiO₂) | 61 | 0 | 0 | 0 | 0 | 0 | 0 |
| Control | None | 20 | 0 | 0 | 0 | 0 | 0 | 0 |

### [Example 4] "Comparison to Organic Substance Having No Iron-reducing Ability"

Reaction products of various organic substances having no iron-reducing ability and iron were prepared and it was investigated whether the products have photocatalytic activities.

### (1) 'Measurement of Photocatalytic Activity'

Iron (III) chloride (FeCl₃) was mixed in an amount of 4 parts by weight in terms of iron element with respect to 100 parts by weight (in terms of dry weight) of ascorbic acid, grape polyphenol, catechin, chlorogenic acid, caffeic acid, or chaff vinegar, and water was added thereto in twice the total weight of the mixture, followed by thermal treatment at 98°C for 1 hour. Thus, reaction products shown in Table 4 were obtained (Samples 4-1 to 4-6). Further, as comparative samples, iron(II) chloride, iron(III) chloride, EDTA iron(III), iron(III) citrate, titanium oxide (TiO₂: anatase-type titanium(IV) oxide, particle size: 100 nm to 300 nm, manufactured by Wako Pure Chemical Industries, Ltd.) were prepared (Comparisons 4-1 to 4-5).

Subsequently, a plurality of aqueous 3.5 ppm basic fuchsin solutions were prepared so that the solutions each contained any one of the samples or the comparative samples at a concentration of 5.5 ppm in terms of iron element (for titanium oxide, in terms of titanium element). The aqueous solutions were allowed to stand still and irradiated with LED visible light (red light: 660 nm, photon density: 100 µmol/m²/sec) for 24 hours while basic fuchsin was quantified with time. Quantification of basic fuchsin and irradiation with LED were carried out in the same manner as in Example 2. Further, as a control, the aqueous solutions were allowed to stand still for 24 hours under dark conditions while basic fuchsin was quantified with time.

Absorbances at 540 nm (results of quantification of basic fuchsin) were measured, and degradation rates were calculated and are shown in Table 4.

The results revealed that, in the case of irradiation with visible light having a wavelength of 660 nm, in the solutions containing the reaction products (samples 4-1 to 4-6) of the various reducing organic substances and iron, basic fuchsin was degraded.

On the other hand, in the solutions containing the various iron compounds or titanium oxide prepared as comparative samples, degradation of basic fuchsin was not observed (Comparisons 4-1 to 4-5). In particular, in the solutions containing the respective complexes of iron and organic substances having no iron-reducing ability, EDTA iron(III) (Comparison 4-3) and iron(III) citrate (Comparison 4-4), degradation of basic fuchsin was not observed.

### (2) 'Discussion'

The results suggested that the iron complexes (reaction products) of "organic substances having no iron-reducing ability" did not exhibit the photocatalytic activity at all. This suggested that the photocatalytic activity of the reaction product of the reducing organic substance and iron was a property specific to the reaction product of the "reducing organic substance having iron-reducing ability" and iron.

**[Table 4]**

| | Reaction product or compound | Rate of degradation by irradiation with light having a wavelength of 660 nm | | | |
|---|---|---|---|---|---|
| | | 6 hr later | 12 hr later | 18 hr later | 24 hr later |
| Sample 4-1 | Ascorbic acid/iron | 8 | 21 | 31 | 57 |
| Sample 4-2 | Grape polyphenol/iron | 19 | 30 | 33 | 70 |
| Sample 4-3 | Catechin/iron | 42 | 52 | 45 | 65 |
| Sample 4-4 | Chlorogenic acid/iron | 46 | 60 | 52 | 73 |
| Sample 4-5 | Caffeic acid/iron | 62 | 72 | 53 | 74 |
| Sample 4-6 | Chaff vinegar component/iron | 26 | 34 | 36 | 64 |
| Comparison 4-1 | Iron(II) chloride | 0 | 0 | 0 | 0 |
| Comparison 4-2 | Iron(III) chloride | 0 | 0 | 0 | 0 |
| Comparison 4-3 | EDTA iron(III) | 0 | 0 | 0 | 0 |
| Comparison 4-4 | Iron(III) citrate | 0 | 0 | 0 | 0 |
| Comparison 4-5 | Titanium oxide (TiO₂) | 0 | 0 | 0 | 0 |
| Control | None | 0 | 0 | 0 | 0 |

### [Example 5] "Sterilization Effect by Irradiation with Visible Light"

The reaction products of the reducing organic substances and iron were used to investigate whether the products were able to sterilize *Escherichia coli* by irradiation with visible light.

### (1) 'Sterilization Test'

Iron(III) chloride (FeCl₃) was mixed in an amount of 4 parts by weight in terms of iron element with respect to 100 parts by weight (in terms of dry weight) of tea dregs (residue of extraction of tea leaves with hot water), and water was added thereto in twice the total weight of the mixture, followed by thermal treatment at 98°C for 1 hour. Thus, a reaction product was obtained. A solid matter obtained by filtration was referred to as "tea leaf component/iron" (Sample 5-1). Further, as a comparative sample, titanium oxide (TiO₂: anatase-type titanium(IV) oxide, particle size: 100 nmto 300 nm, manufactured by Wako Pure Chemical Industries, Ltd.) (Comparison 5-1) was prepared.

The samples were added to and mixed in suspensions of 10⁶ cfu/mL *Escherichia coli* (ATCC1124) at concentrations in terms of iron element (for titanium oxide, in terms of titanium element) shown in Table 5, and the suspensions were irradiated with visible light (blue light: 470 nm, photon density: 50 µmol/m²/sec) for 24 hours. After that, the suspensions were applied to *Escherichia coli* assay plates, and viable *Escherichia coli* counts were determined. Further, as a control, only *Escherichia coli* was applied in the same way as above and subjected to the same experiment as above. The results are shown in FIG. 1. Further, the presence or absence of sterilization ability was evaluated on a two-point scale ("+": having sterilization ability, having no sterilization ability), and the results are shown in Table 5.

The results revealed that *Escherichia coli* was able to be killed by adding the tea leaf component/iron (solid matter after filtration) at a concentration of 20 ppm or more in terms of iron element and irradiating the sample with visible light (Sample 5-1) .

On the other hand, even when titanium oxide used as a comparative sample was added at high concentrations, no decrease in *Escherichia coli* by irradiation with visible light was able to be observed (Comparison 5-1).

### (2) 'Discussion'

The results revealed that the sample containing the reaction product of the reducing organic substance having iron-reducing ability and iron exhibited a sufficient sterilization effect provided by the photocatalytic activity when irradiated with only visible light. Further, it was found that even the sample containing the reaction product at a concentration of 20 ppm sufficiently exhibited the sterilization effect. The results also revealed that the solid part in the reaction solution had a strong photocatalytic activity (in Example 2, the filtrate of the reaction solution was found to have a stronger activity).

On the other hand, the results revealed that the sample containing titanium oxide exhibited no photocatalytic activity and provided no sterilization effect when irradiated with only visible light.

**[Table 5]**

| Reaction product or compound | | Sterilization effect | | | |
|---|---|---|---|---|---|
| | | 0 ppm (Control) | 20 ppm | 40 ppm | 80 ppm |
| Sample 5-1 | Tea leaf component/iron | - | + | + | + |
| Comparison 5-1 | Titanium oxide (TiO₂) | | - | - | - |

### [Example 6] "Sterilization Effect by Irradiation with Sunlight"

The reaction products of the reducing organic substances and iron were used to investigate whether the products were able to sterilize *Escherichia coli* by irradiation with sunlight.

### (1) 'Sterilization Test'

Iron (III) chloride (FeCl₃) was mixed in an amount of 4 parts by weight in terms of iron element with respect to 100 parts by weight (in terms of dry weight) of tea dregs (residue of extraction of tea leaves with hot water) or coffee grounds (residue of extraction of roasted and ground coffee bean with hot water), and water was added thereto in twice the total weight of the mixture, followed by thermal treatment at 98°C for 1 hour. Thus, a reaction product was obtained. A solid matter obtained by filtration was referred to as "tea leaf component/iron" (Sample 6-1) or "roasted coffee bean component/iron" (Sample 6-2). Further, as a comparative sample, titanium oxide (TiO₂: anatase-type titanium(IV) oxide, particle size: 100 nm to 300 nm, manufactured by Wako Pure Chemical Industries, Ltd.) (Comparison 6-1) was prepared.

The samples shown in Table 6 were added to and mixed in suspensions (10⁶ cfu/mL) of *Escherichia coli* (ATCC1124) at a concentration of 5.5 ppm in terms of iron element (for titanium oxide, in terms of titanium element), and the suspensions were irradiated with sunlight (irradiance: 763 W/m², UV(A+B) 3.28 mW/cm², photon density: 1,514 µmol/m²/sec) for 10 minutes. After that, the suspensions were applied to *Escherichia coli* assay plates, and viable *Escherichia coli* counts were determined. Further, as a control, only *Escherichia coli* was applied in the same way as above and subjected to the same experiment as above. The results are shown in FIG. 2. Further, the presence or absence of sterilization ability was evaluated on a two-point scale ("+": having sterilization ability, "-": having no sterilization ability), and the results are shown in Table 6.

The results revealed that *Escherichia coli* was able to be killed by adding the reaction product, i.e., the tea leaf component/iron (solid matter after filtration) or the roasted coffee bean component/iron (solid matter after filtration) and irradiating the sample with sunlight (Samples 6-1 and 6-2). The results revealed that the sterilization effect provided a sufficient effect through addition of the reaction product at a very low concentration of 5.5 ppm in terms of iron element. The results also revealed that a sufficient effect was provided even when the product was irradiated with sunlight in a very short time, as short as 10 minutes.

On the other hand, when the comparative sample containing titanium oxide was irradiated with sunlight under the same conditions as those for Samples 2-1 and 2-2, a large amount of *Escherichia coli* survived (Comparison 6-1).

### (2) 'Discussion'

The results revealed that the sample containing the reaction product of the reducing organic substance and iron exhibited a sterilization effect provided by the very strong photocatalytic activity when irradiated with sunlight. Further, it was found that even the sample containing the reaction product at a very low concentration of 5.5 ppm exhibited a sufficient sterilization effect even when irradiated with sunlight for 10 minutes.

On the other hand, the results revealed that the sample containing titanium oxide did not sufficiently exhibit the photocatalytic activity and provided an insufficient sterilization effect when irradiated with only sunlight for a short time.

**[Table 6]**

| | Reaction product or compound | Sterilization ability |
|---|---|---|
| Sample 6-1 | Tea leaf component/iron 5.5 ppm | + |
| Sample 6-2 | Roasted coffee bean component/iron 5.5 ppm | + |
| Comparison 6-1 | Titanium oxide (TiO₂) 5.5 ppm | - |
| Control | None | - |

### [Example 7] "Sterilization Effect by Plurality of Times of Continuous Irradiation with Sunlight"

The reaction product of the reducing organic substance and iron was used to investigate whether the product was able to sterilize *Escherichia coli* by a plurality of times of continuous irradiation with sunlight.

### (1) 'Continuous Sterilization Test'

30 mg of the roasted coffee bean component/iron prepared in Example 6 (Sample 6-1: solid matter after filtration) was added to and mixed in 300 mL of sterilized water, and the mixture was poured into a sealable PET bottle. 90 µL of a suspension of *Escherichia coli* (ATCC1124) (3.5×10⁸ cfu/mL) was added to the PET bottle and irradiated with sunlight for 10 minutes. 1 mL of the bacterial solution was collected after irradiation.

1 Hour after the collection, the *Escherichia coli* suspension was added again in the same volume as above to the PET bottle and subjected to the second irradiation with sunlight for 10 minutes, and 1 mL of the bacterial solution was collected after irradiation. Further, 1 hour after the collection, the *Escherichia coli* suspension was added again in the same volume as above to the PET bottle and subjected to the third irradiation with sunlight for 10 minutes, and 1 mL of the bacterial solution was collected after irradiation.

The bacterial solutions collected were applied to *Escherichia coli* assay plates, and viable *Escherichia coli* counts were determined. Further, as a control, only *Escherichia coli* was applied in the same way as above and subjected to the same experiment as above. The results are shown in FIG. 3. Further, the presence or absence of sterilization ability was evaluated on a two-point scale ("+": having sterilization ability, "-" : having no sterilization ability), and the results are shown in Table 7.

The results revealed that, in the solution containing the roasted coffee bean component/iron as the reaction product, *Escherichia coli* was able to be sterilized continuously three times only by re-irradiation with light even after sterilization by the photocatalytic activity was once performed. Further, in all of the samples after the first to third sterilization, *Escherichia coli* was killed completely, and hence decreases in potency of the photocatalytic activity owing to continuous use were not observed.

### (2) 'Discussion'

The results revealed that the photocatalytic activity of the reaction product of the reducing organic substance and iron was not lost by one photocatalytic reaction. That is, the results revealed that the reaction product was a substance that can be used repeatedly and stably for a long time as photocatalytic activity.

It should be noted that the property was considered as a property resulting from the fact that the reaction product (Fe²⁺ complex of the reducing organic substance) had a stable structure.

**[Table 7]**

| | Reaction product | Sterilization ability | | |
|---|---|---|---|---|
| | | First | Second | Third |
| Sample 6-1 | Roasted coffee bean component/iron | + | + | + |
| Control | None | - | - | - |

### Industrial Applicability

The photocatalyst of the present invention is expected to be widely used for sterilization or for degradation of organic substances in wide fields of food, medicine, public health, agriculture, environmental cleanup, and the like.

## Claims

1. A photocatalyst, comprising, as an active component, a reaction product obtained by mixing a reducing organic substance having iron-reducing ability or a feedstock for supplying the reducing organic substance with an iron-supplying source in the presence of water.

2. A photocatalyst according to claim 1, wherein the reducing organic substance comprises a polyphenol and/or ascorbic acid.

3. A photocatalyst according to claim 2, wherein the polyphenol comprises one or more compounds selected from the group consisting of chlorogenic acid, caffeic acid, tannic acid, and catechin, or a compound having one or more of the compounds selected from the group consisting of chlorogenic acid, caffeic acid, tannic acid, and catechin in its molecule.

4. A photocatalyst according to any one of claims 1 to 3, wherein the feedstock for supplying the reducing organic substance comprises one or more plant bodies selected from the group consisting of a fruit, a seed, a stem and leaf, a bud, a flower, a root, and an underground stem, or a processed product of the plant body.

5. A photocatalyst according to any one of claims 1 to 4, wherein the feedstock for supplying the reducing organic substance comprises roasted coffee beans, tea leaves, a squeezed fruit juice, or a plant dry distillation liquid.

6. A photocatalyst according to any one of claims 1 to 5, wherein the iron-supplying source is mixed so that the content of the iron-supplying source is from 0.1 part by weight to 10 parts by weight, in terms of weight of iron element, with respect to 100 parts by weight, in terms of dry weight, of the reducing organic substance or the feedstock for supplying the reducing organic substance.

7. A photocatalyst according to any one of claims 1 to 6, wherein the reaction product exhibits a photocatalytic activity when irradiated with light having a wavelength of ultraviolet light, visible light, or infrared light.

8. A photocatalyst according to any one of claims 1 to 7, wherein the photocatalyst exhibits an organic substance degradation action by the photocatalytic activity.

9. An organic substance decomposer, comprising the photocatalyst of any one of claims 1 to 8.

10. An organic substance degradation method, comprising:
bringing the photocatalyst of any one of claims 1 to 8 into contact with an object to be degraded; and
irradiating the photocatalyst with light having a wavelength of ultraviolet light, visible light, or infrared light.

11. A sanitizer, comprising the photocatalyst of any one of claims 1 to 8.

12. A sterilization method, comprising:
bringing the photocatalyst of any one of claims 1 to 8 into contact with an object to be sterilized; and
irradiating the photocatalyst with light having a wavelength of ultraviolet light, visible light, or infrared light.
